Europäisches Patentamt

European Patent Office

Office européen des brevets

(19)

(11) **EP 0 821 935 A2**

(12) **EUROPÄISCHE PATENTANMELDUNG**

(43) Veröffentlichungstag:
**04.02.1998 Patentblatt 1998/06**

(51) Int. Cl.$^6$: **A61K 7/13**, A61K 7/06

(21) Anmeldenummer: 97110979.8

(22) Anmeldetag: **02.07.1997**

(84) Benannte Vertragsstaaten:
**AT BE CH DE DK ES FI FR GB GR IE IT LI LU MC NL PT SE**

(30) Priorität: **01.08.1996 DE 19631035**

(71) Anmelder:
**Wella Aktiengesellschaft
64274 Darmstadt (DE)**

(72) Erfinder:
• **Lenz, Uwe, Dr.
64673 Zwingenberg (DE)**
• **Schollmeyer, Claudia
64291 Darmstadt (DE)**

(54) **Mittel zum Färben und Tönen von Haaren**

(57)     Gegenstand der Erfindung ist ein Mittel zum Färben und Tönen von Haaren, welches einen sauren pH-Wert aufweist und eine Kombination aus einem nichtionischen Polymer und einem Proteinhydrolysat und/oder einer Aminosäure enthält.

EP 0 821 935 A2

## Beschreibung

Die vorliegende Erfindung betrifft ein Mittel zum Färben beziehungsweise Tönen von menschlichen Haaren mit verbesserter Färbeintensität, das gleichzeitig eine konditionierende Wirkung aufweist.

Es ist allgemein bekannt, daß Haarfärbemittel in zwei Kategorien aufgeteilt werden, nämlich einerseits die permanenten Haarfärbemittel, die grundsätzlich Haarfarbstoffvorprodukte enthalten, die zusammen mit Oxidationsmitteln je nach Zusammensetzung die gewünschte Färbung auf dem Haar entwickeln; und andererseits semipermanente Haarfärbemittel, die direktziehende Farbstoffe enthalten, die zur Entwicklung ihrer Färbeleistung keinerlei Oxidationsmittelzusatzes bedürfen. Dementsprechend sind die Färbungen auch weniger dauerhaft und weniger intensiv als die mit Permanentfarbstoffen erzielbaren Färbungen.

Diese Farbzusammensetzungen auf Basis direktziehender Farbstoffe werden in der Regel entweder als Tönungsshampoos, als Tönungslotionen oder als Tönungsfestiger, gegebenenfalls auch als Aerosolschaum, appliziert. Diese Zusammensetzungen enthalten auch haarkonditionierende Polymere, insbesondere kationische Polymere. Sie weisen in der Regel einen pH-Wert von mehr als 7 auf. Die mit diesen Zusammensetzungen erzielbare Farbintensität ist jedoch nicht befriedigend; ebenso wird, bedingt durch den alkalischen pH-Wert, auch keine befriedigende Konditionierung des Haares erzielt.

Es bestand daher ein Bedürfnis nach Haarfärbeprodukten auf Basis direktziehender Farbstoffe, die die genannten Nachteile nicht aufweisen.

Überraschenderweise wurde nunmehr gefunden, daß ein nichtoxidatives Haarfärbemittel, welches einen sauren pH-Wert aufweist und eine Kombination von nichtionischen Polymeren und Proteinhydrolysaten und/oder Aminosäuren enthält, diese Aufgabe in hervorragender Weise löst.

Gegenstand der vorliegenden Anmeldung ist daher ein Mittel zum Färben und Tönen von Haaren, insbesondere menschlichen Haaren, auf der Basis von direktziehenden Farbstoffen, welches einen sauren pH-Wert aufweist und und dadurch gekennzeichnet ist, daß es a) mindestens ein nichtionisches Polymer und b) mindestens ein Proteinhydrolysat und/oder mindestens eine Aminosäure enthält.

Das erfindungsgemäße Mittel enthält mindestens einen direktziehenden Farbstoff, wobei die Gesamtmenge der direktziehenden Farbstoffe vorzugsweise 0,01 bis 5 Gewichtsprozent, insbesondere 0,05 bis 3 Gewichtsprozent, beträgt.

Wesentlich ist, daß das erfindungsgemäße Mittel einen sauren pH-Wert, das heißt einen pH-Wert von 2 bis 7, insbesondere 3 bis 5, aufweist.

Als direktziehende Farbstoffe können übliche, physiologisch unbedenkliche, direktziehende Farbstoffe aus der Gruppe der Nitrofarbstoffe, Azofarbstoffe, Chinonfarbstoffe und Triphenylmethanfarbstoffe, wie zum Beispiel 4,N-Ethyl,N-(2'-hydroxyethyl)amino-1-(2''-hydroxyethyl)amino-2-nitro-benzol, 1-Amino-3-methyl-4-(2'-hydroxyethyl)amino-6-nitro-benzol, 2,2'-[(Amino-3-nitrophenyl)imino]-bisethanol Hydrochlorid (HC Red 13), 1-(2'-Hydroxyethyl)amino-2-nitro-4-bis-(2''-hydroxyethyl)amino-benzol, 4-Bis-(2'-Hydroxyethyl)amino-1-(2''-methoxyethyl)amino-nitrobenzol, 1-(2',3'-Dihydroxypropyl)amino-2-nitro-4-[methyl-(2''-hydroxyethyl)amino]-benzol, 1-[(2',3'-Dihydroxypropyl)amino]-2-nitro-4-[ethyl-2''-(hydroxyethyl)amino]-benzol, 1-(3'-Hydroxypropylamino)-2-nitro-4-bis-(2''-hydroxyethylamino)-benzol, 1-Amino-4-(2'-hydroxyethyl)-amino-nitrobenzol, 1-Hydroxy-2-amino-4,6-dinitro-benzol, 1-Amino-2-nitro-4-bis-(2'-hydroxyethyl)amino-benzol, 1-Amino-2-nitro-4-(2'-hydroxyethyl)amino-5-chlorbenzol, 1-(2'-Hydroxyethyl)amino-2-nitro-4-amino-benzol, 1-Hydroxy-3-nitro-4-amino-benzol, 1-(2'-Aminoethyl)amino-2-nitro-4-(2''-hydroxyethyl)-oxybenzol, 3-Nitro-4-(2'-hydroxyethyl)amino-phenylglycerinether, 1-Amino-5-chlor-4-(2',3'-dihydroxypropyl)amino-2-nitrobenzol, 1,4-Bis-[(2',3'-dihydroxypropyl)amino]-5-chlor-2-nitro-benzol, 1-Hydroxy-2-(2'hydroxyethyl)amino-4,6-dinitro-benzol, 2-Amino-6-chlor-4-nitrophenol, 1-Hydroxy-3-nitro-4-(3'-hydroxypropylamino)-benzol, 3-Nitro-4-ethylamino-benzoesäure, 4-Amino-2-nitro-diphenylamino-2-carbonsäure, 2-Chlor-6-ethylamino-4-nitrophenol, 2,5-Diamino-6-nitropyridin, 1,2,3,4-Tetrahydro-6-nitrochinoxalin, 4-(2'-Hydroxyethyl)amino-3-nitro-benzonitril, 4-(2'-Hydroxyethyl)amino-3-nitro-benzamid, 1-Amino-2-(2'-hydroxyethyl)amino-5-nitro-benzol, 1-Methoxy-2-(2'-hydroxyethyl)amino-5-nitro-benzol, 1-Hydroxy-3-nitro-4-(2'-hydroxyethyl)amino-benzol, 1-Hydroxy-2-amino-3-nitro-benzol, 1-Amino-2-methyl-6-nitro-benzol, 1-(2'Hydroxyethyl)-oxy-3-methylamino-4-nitro-benzol, 1-Methylamino-2-nitro-5-(2',3'-dihydroxypropyl)-oxybenzol, 1-(2'-Hydroxyethyl)amino-2-hydroxy-4-nitro-benzol, 1-Methoxy-3-(2'-aminoethyl)amino-4-nitro-benzol, 1-(2'-Ureidoethyl)amino-4-nitro-benzol, 1-(2'-Hydroxyethyl)amino-2-nitro-benzol, 4-(2'-Hydroxyethyl)amino-3-nitro-trifluormethyl-benzol, 2,4-Bis-[N-(2'-hydroxyethyl)amino]-5-chlor-nitrobenzol, 4-(2',3'-Dihydroxypropyl)amino-3-nitro-trifluormethyl- benzol, 4-(2'-Hydroxymethyl)amino-3-nitro-methylbenzol, 4-(2'-Hydroxyethyl)amino-3-nitro-chlorbenzol, 1-(4'-Nitrophenylazo)-2-methyl-4-bis-(2''-hydroxyethyl)amino-benzol, 1-(3'-Nitro-4-amino)-phenylazo-2-hydroxy-7-trimethyl-ammoniumchlorid-naphthalin, 1-(2'Hydroxy-4'sulfo-6'nitro)-naphthylazo-2-hydroxynaphthalin, 1-(4'-Aminophenylazo)-2-methyl-4-bis-[(2'-hydroxyethyl)-amino]-benzol, 5-(4'-Dimethylaminophenylazo)-1,4-dimethyl-triazoniumchlorid, 1-(2'-Methoxyphenylazo)-2-hydroxy-7-trimethylammonium-naphthalinchorid, 1-(4'-Aminophenylazo)-2-hydroxy-7-trimethylammonium-naphthalin, 4-(3'-Trimethylammoniumphenylazo)-N-phenyl-3-methyl-pyrazolon(5), 4-Hydroxy-3-[(4'-sulfo-1'-naphthyl)azo]-1-naphthalinsulfonsäure, 1-(4'-Sulfophenylazo)-2-hydroxynaphthalin, 1-(4'-Sulfonphenylazo)-2-hydroxy-6-sulfo-naphthalin, 4-Amino-[4'-bis-(2''-hydroxyethyl)amino]-azobenzol, 4-Amino-[4'-bis-(2''-hydroxyethyl)amino]-2'-methyl-azobenzol, 3-(2',6'-Diaminopyridyl-3'-azo)-pyridin, 7-Phenylazo-1-amino-3,6-disulfo-8-hydroxy-naphthalin, 5-Acetylamino-4-hydroxy-3-[(2'-methylphenyl)azo]-2,7-naphthalindisulfonsäure, 2-(2',4'-Dimethylphenylazo)-6-(4''-sulfophenylazo)-1,3-dihydroxybenzol, 1,4-Bis-(2',3'-dihydroxypropyl)amino-anthrachinon, 1-Methylamino-4-(2'-hydroxyethyl)amino-anthrachinon, 2-(2'-Aminoethyl)amino-anthrachinon, 2-Brom-4,8-diamino-6-(3'-trimethylammonium)-phenylamino-1,5-naphthochinon, 1-(2'-Sulfo-4'-methyl-phenyl)-amino-4-hydroxy-anthrachinon, 1,4-Diamino-anthrachinon, 1-Amino-2-sulfo-4-cyclohexylamino-anthrachinon, 1-Methylamino-4-aminopropylamino-anthrachinon, 1-Aminopropylamino-anthrachinon, 4',4'',4'''-Triamino-3-methyl-triphenyl-carboniumchlorid, Bis-(4,4-Diethylaminophenyl)-4'-ethylamino-naphthyl-carboniumchlorid, Bis-(4,4-Dimethylaminophen)-4'-phenylamino-naphthyl-carbonsiumchlorid und 4,4-Bis-(N-Ethyl-3-sulfobenzyl)amino-2''-sulfofuchsonium, 2,4-Dinitro-1-naphthol-7-sulfonsäure-Dinatriumsalz (Acid Yellow 1; CI 10 316); 2-(2'-Chinolyl)-1H-indene-1,3(2H)-dion-monodisulfonsäure-Dinatriumsalz (Acid Yellow 3; CI 47 005); 4,5-Dihydro-5-oxo-1-(4'-sulfophenyl)-4-[(4''-sulfophenyl)azo]-1H-pyrazol-3-carbonsäure-Trinatriumsalz (Acid Yellow 23; CI 19 140); 3',6'-Dihydroxyspiro [isobenzofuran-1(3H), 9'(9H)-xanthen]-3-on (Acid Yellow 73; CI 45 350:1); 5-[2',4,-Dinitrophenyl)amino]-2-(phenylamino)-benzolsulfonsäure-Natriumsalz (Acid Orange 3; CI 10 385); 4-[(2',4'-Dihydroxyphenyl)azo]-benzolsulfonsäure-Natrium-salz (Acid Orange 6; CI 14 270); 4-[2'-Hydroxy-1'-naphthyl)azo]-benzolsulfonsäure-Natriumsalz (Acid Orange 7; CI 15 510); 4-[[3'-[(2'',4''-Dimethylphenyl)azo]-2',4'-dihydroxyphenyl]azo]-benzolsulfonsäure-Natriumsalz (Acid Orange 24;CI 20 170); 4-Hydroxy-3-[(4'-sulfo-1'-naphthyl)azo]-1-naphthalinsulfonsäure-Dinatriumsalz (Acid Red 14; CI 14 720); 7-Hydroxy-8-[(4'-sulfo-1'-naphthyl)azo]-1,3-naphthalindisulfonsäure-Trinatriumsalz (Acid Red 18; CI 16 255); 3-Hydroxy-4-[(4'-sulfo-1'-naphthyl)azo]-2,7-naphthalindisulfonsäure-Trinatriumsalz (Acid Red 27; CI 16 185); 5-Amino-4-hydroxy-3-phenylazo-2,7-naphtalindisulfonsäure-Dinatriumsalz (Acid Red 33; CI 17 200); 5-(Acetylamino)-4-hydroxy-3-[(2'-methylphenyl)azo]-2,7-naphthalindisulfonsäure-Dinatriumsalz (Acid Red 35; CI 18 065); 3',6'-Dihydroxy-2',4',5',7'-tetraiodospiro-[isobenzofuran-1(3H), 9'(9H)-xanthen]-3-on-Dinatriumsalz (Acid Red 51; CI 45 430); 3,6-Bis-(diethylamino)-9-(2', 4'-disulfophenyl)-xanthyliumhydroxid-Natriumslz (Acid Red 52; CI 45 100); 7-Hydroxy-8-[[4'-(phenylazo)phenyl]azo]-1,3-naphthalindisulfonsäure-Dinatriumsalz (Acid Red 73; CI 27 290); 2',4',5',7'-Tetrabromo-3',6'-dihydroxyspiro[isobenzofuran-1(3H),9'(9H)-xanthen]-3-on-Dinatriumsalz (Acid Red 87; CI 45 380);2',4',5',7'-Tetrabromo-4,5,6,7-tetrachloro-3',6'-dihydroxyspiro[isobenzofuran-1(3H),9'(9H)-xanthen]-3-on-Dinatriumsalz (Acid Red 92; CI 45 410); 3',6'-Dihydroxy-4',5'-dijodospiro[isobenzofuran-1(3H),9'(9H)-xanthen]-3-on-Dinatriumsalz (Acid Red 95; CI 45 425); Acid Red 195; Acid Blue 9 (CI 42 090); 2,2'-[(9,10-Dihydro-9,10-dioxo-1,4-anthracendiyl)diimino]-bis-(5-methyl-benzolsulfonsäue)-Dinatriumsalz (Acid Green 25; CI 61 570); N-[4-[[4'-(Dimethylamino)phenyl]-(2''-hydroxy-3'',6''-disulfo-1''-naphthyl)-methylen]-2,5-cyclohexadien-1-yliden]-N-methylmethanaminiumhydroxid (Acid Green 50; CI 44 090); N-[4-[[4'-Diethylamino)phenyl]-(2'',4''-disulfophenyl)-methylen]-2,5-cyclohexadien-1-yliden]-N-ethylethanaminiumhydroxid-Natriumsalz (Acid Blue 1; CI 42 045); N-[4-[[4'-Diethylamino)phenyl]-(5''-hydroxy-2'',4''-disulfo-phenyl)-methylen]-2,5-caclohexadien-1-yliden]-N-ethyl-ethanaminiumhydroxid-Calciumsalz (Acid Blue 3; CI 42 051); 1-Amino-4-(cyclohexylamino)-9,10-dihydro-9,10-dioxo-2-anthracensulfonsäure-Natriumsalz (Acid Blue 62; CI 62 045); 2-(1',3'-Dihydro-3'-oxo-5'-sulfo-2'H-indol-2'-yliden)-2,3-dihydro-3-oxo-1H-indol-5-sulfonsäure-Dinatriumsalz (Acid Blue 74; CI 73 015);9-(2'-Carboxyphenyl)-3-[(2''-methylphenyl)amino]-6-[(2'''-methyl-4'''-sulfophenyl)amino]]-xanthylium-hydroxid-Natriumsalz (Acid Violet 9; CI 45 190); 2-[(9',10'-Dihydro-4'-hydroxy-9',10'-dioxo-1'-anthracenyl)-amino]-5-methylbenzolsulfonsäure-Natriumsalz (Acid Violet 43; CI 60 730); 3,3'-[Sulfonyl-bis(2-nitro-4,1-phenylen)imino]-bis-[6-phenylamino)-benzol-dinatriumsulfonat] (Acid Brown 13; CI 10 410); 4-Amino-5-hydroxy-3-[(4,-nitrophenyl)azo]-6-(phenylazo)-2,7-naphthalindisulfonsäure-Dinatriumsalz (Acid Black 1; CI 20 470); 3-Hydroxy-4-[(2'-hydroxy-1'-naphthyl)azo]-7-nitro-1-naphthalinsulfonsäure-Natriumsalz (Acid Black 52; CI 15 711); N-[4-[[4'-(Diethylamino)phenyl]-[4''-(ethylamino)-1''-naphthyl]-methylen]-2,5-cyclohexadien-1-yliden]-N-ethyl-ethanammoniumchlorid (Basic Blue 7; CI 42 595); 4-[(4'-Aminophenyl)-(4'-imino-2',5'-cyclohexadien-1'-yliden)-methyl]-2-methyl-aminobenzol-Hydrochlorid (Basic Violet 14; CI 42 510); 4-(Acetylamino)-5-hydroxy-6-[[7'-sulfo-4'-[(4''-Sulfophenyl)azo]-1'-naphthyl]azo]-1,7-naphthalin-disulfonsäure-Tetranatriumsalz (Brilliant Black 1; CI 28 440); [8-(p-Aminophenyl)azo]-7-hydroxy-2-naphthyl]-trimethylammoniumchlorid (Basic Brown 16; CI 12 250); [8-[4'-Amino-2'-nitrophenyl)azo]-7-hydroxy-2-naphthyl]-trimethylammoniumchlorid (Basic Brown 17; CI 12 251); 7-Hydroxy-8-[(2'-methoxyphenyl)azo]-N,N,N-trimethyl-2-naphthylammoniumchlorid (Basic Red 76; CI 12 245) und 3-[(4'-Amino-6'-bromo-5',8'-dihydro-1'-hydroxy-8'-imino-5'-oxo-2'-naphthyl)amino]-N,N,N- trimethylammonium-chlorid (Basic Blue 99; CI 56 059), verwendet werden.

Als Proteinhydrolysat wird vorzugsweise Keratinhydrolysat, Seidenproteinhydrolysat oder eine Mischung dieser beiden Verbindungen eingesetzt, wobei die Einsatzmenge vorzugsweise 0,01 bis 5 Gewichtsprozent beträgt.

Als Aminosäure können sowohl neutrale als auch saure oder basische Aminosäuren verwendet werden. Beispiele für geeignete Aminosäuren sind Glycin, Alanin, Valin, Leucin, Glutaminsäure und Arginin.

Die Gesamtmenge an Aminosäuren beträgt in dem erfindungsgemäßen Mittel vorzugsweise 0,01 bis 5 Gewichtsprozent.

Als geeignete nichtionische Polymere sind insbesondere Vinylpyrrolidon-Homo- und Copolymerisate zu nennen, wobei Polyvinylpyrrolidon besonders bevorzugt ist.

Die Einsatzmenge des nichtionischen Polymers beträgt vorzugsweise 0,1 bis 10 Gewichtsprozent, wobei eine Menge von 0,2 bis 3 Gewichtsprozent besonders bevorzugt wird.

Das erfindungsgemäße Haarfärbemittel enthält weiterhin zur pH-Werteinstellung organische oder anorganische Säuren, wie zum Beispiel Essigsäure, Zitronensäure, Weinsäure, Salzsäure oder Phosphorsäure.

Weiterhin können die erfindungsgemäßen Haarfärbemittel alle für derartige Mittel bekannten und üblichen Zusätze enthalten, deren Art und Charakter von der Applikationsform des Mittels abhängig ist. Es sind diese Fette und Wachse, Fettalkohole, Tenside und Emulgatoren, Puffersubstanzen, Lösungsmittel, Lösungsvermittler, Konservierungsmittel, Parfüms usw.

Als besonders vorteilhaft hat sich hierbei der Zusatz von kationischen Tensiden, zum Beispiel Alkyltrimethylammoniumchloriden oder -bromiden, erwiesen.

Die erfindungsgemäßen Farbkonditionierungsmittel liegen als Emulsion, Dispersion oder (gegebenenfalls verdickte) Lösung vor und können auch als Aerosolschaum konfektioniert werden. Diese Zusammensetzungen und ihre Herstellung sind dem Fachmann grundsätzlich bekannt und bedürfen daher keiner näheren Erläuterung.

Die erfindungsgemäßen Farbkonditionierungsmittel sind vorzugsweise frei von kationischen Polymeren.

Die erfindungsgemäßen Mittel gelangen je nach gewünschter Farbintensität in unverdünnter Form oder nach einer 1:1- bis 1:50-Verdünnung mit Wasser zur Anwendung.

Das Haarfärbemittel wird auf das gewaschene und handtuchtrockene Haar in einer für die Haarfärbung ausreichenden Menge aufgetragen und sodann einwirken gelassen. Anschließend wird das Haar getrocknet.

Es ist jedoch auch möglich, das Haarfärbemittel nach einer Einwirkungszeit von 1 bis 20 Minuten, gegebenenfalls unter Wärmeeinwirkung (30 bis 50° Celsius), mit Wasser auszuspülen und anschließend zu trocknen.

Das erfindungsgemäße Haarfärbemittel ermöglicht intensive und schonende Haarfärbungen bei gleichzeitiger Konditionierung und Festigung der Haare.

Die nachfolgenden Beispiele sollen den Gegenstand der vorliegenden Erfindung näher erläutern ohne diesen hierauf zu beschränken.

## Beispiele

## Haartönungsmittel

| | |
|---|---|
| 1,0 g | Polyvinylpyrrolidon (Luviskol® K 90 der Firma BASF/Ludwigshafen, BRD) |
| 1,0 g | Cetyltrimenthylammoniumchlorid |
| 0,5 g | Keratinhydrolysat |
| 0,5 g | Parfüm |
| 0,1 g | Glycin |
| 0,1 g | HC Red 13 |
| 0,1 g | Acid Violet 43 (CI 60 730) |
| 96,7 g | Wasser |
| 100,0 g | |

Der pH-Wert beträgt 4,1. Das Haarfärbemittel ergibt eine violett-rosé Farbe.

Alle Prozentangaben stellen, soweit nicht anders angegeben, Gewichtsprozente dar.

## Patentansprüche

1. Mittel zum Färben und Tönen von Haaren auf der Basis von direktziehenden Farbstoffen, welches einen sauren pH-Wert aufweist, dadurch gekennzeichnet, daß es a) mindestens ein nichtionisches Polymer und b) mindestens ein Proteinhydrolysat und/oder mindestens eine Aminosäure enthält.

2. Mittel nach Anspruch 1, dadurch gekennzeichnet, daß es einen pH-Wert von 2 bis 7 aufweist.

3. Mittel nach Anspruch 1 oder 2, dadurch gekennzeichnet, daß die direktziehenden Farbstoffe in einer Gesamtmenge von 0,01 bis 5 Gewichtsprozent enthalten sind.

4. Mittel nach einem der Ansprüche 1 bis 3, dadurch gekennzeichnet, daß als nichtionisches Polymer Polyvinylpyrrolidon verwendet wird.

5. Mittel nach einem der Ansprüche 1 bis 4, dadurch gekennzeichnet, daß die Gesamtmenge an nichtionischem Polymer 0,01 bis 10 Gewichtsprozent beträgt.

6. Mittel nach einem der Ansprüche 1 bis 5, dadurch gekennzeichnet, daß das Proteinhydrolysalysat ausgewählt ist aus Keratinhydrolysat, Seidenproteinhydrolysat und Mischungen dieser beiden Verbindungen.

7. Mittel nach einem der Ansprüche 1 bis 6, dadurch gekennzeichnet, daß es das Proteinhydrolysat in einer Menge von 0,01 bis 5 Gewichtsprozent enthält.

8. Mittel nach einem der Ansprüche 1 bis 7, dadurch gekennzeichnet, daß die Aminosäure ausgewählt ist aus Glycin, Alanin, Valin, Leucin, Glutaminsäure und Arginin.

9. Mittel nach einem der Ansprüche 1 bis 8, dadurch gekennzeichnet, daß es die Aminosäure in einer Gesamtmenge von 0,01 bis 5 Gewichtsprozent enthält.

10. Mittel nach einem der Ansprüche 1 bis 9, dadurch gekennzeichnet, daß es ein kationisches Tensid enthält.